# EUROPEAN PATENT APPLICATION

(11) **EP 4 537 654 A1**
(43) Date of publication of application: **16.04.2025**
(21) Application number: 24206161.2
(22) Date of filing: 11.10.2024
(51) Int. Cl.: A01G 7/04, A01G 22/05, A01H 1/02, A01M 1/04

(54) **SYSTEMS FOR DETERMINING POLLINATION ILLUMINATION PATTERNS AND ASSISTING POLLINATION IN CONTROLLED ENVIRONMENT AGRICULTURE AND RELATED METHODS**

(30) Priority: 12.10.2023 US 202363589796 P; 12.10.2023 US 202363589807 P
(71) Applicant: Sollum Technologies Inc., Montréal, Québec H3C 3X6 (CA)
(72) Inventor: ROY-MOISAN, François, Montreal, H3C 3X6 (CA); BRUN, Louis, Montreal, H3C 3X6 (CA); TREMBLAY, Marc, Montreal, H3C 3X6 (CA)
(74) Representative: Lavoix

(57) **Abstract**

There is provided a method for determining pollination illumination patterns within a horticultural structure, including defining a control zone associated with a first light source, the first light source being configured to illuminate a control plant or crop according to a control lighting scenario; defining a test zone associated with a second light source, the second light source being configured to illuminate a test plant or crop according to a test lighting scenario; illuminating the control plant or crop with the first light source according to the control lighting scenario; illuminating the test plant or crop with the second light source according to the test lighting scenario in the test zone; and following a release of pollinators, determining whether the test lighting scenario impacts a pollination-related property of the test plant or crop with respect to a pollination-related property of the control plant or crop.

## Description

### TECHNICAL FIELD

The technical field generally relates to lighting, and more particularly concerns techniques for determining pollination illumination patterns, as well as assisting or promoting pollination, in controlled agricultural environment.

### BACKGROUND

Tomatoes, peppers, eggplants, and seed crops (e.g., cabbage, carrots, and cucumbers) are some examples of plants or crops which require pollination during their growth. These plants or crops can grow in horticultural structures such as, for example, greenhouse. However, options for pollinating plants or crops in the context of controlled environment agriculture are often limited. Therefore, it remains challenging to develop and use techniques that could potentially help optimizing or assisting the pollination process of plants or crops growing within a horticultural structure.

There is a need for techniques that can provide improvements in methods and systems assisting the pollination process in controlled environment agriculture.

### SUMMARY

The present techniques generally concern an automated system enabling the experimental discovery and classification of static and dynamic light patterns capable of attracting flying insects toward a specific location within the controlled agriculture environment, retaining insects at a specific location within the controlled environment, stimulating their target activity at a specific location and repulsing flying insects from a specific location.

In accordance with one aspect, there is provided method for determining pollination illumination patterns within a horticultural structure. The method includes defining a control zone within the horticultural structure, the control zone being associated with a first horticultural light source, the first horticultural light source being configured to illuminate a control plant or crop according to a control lighting scenario; defining a test zone within the horticultural structure, the test zone being associated with a second horticultural light source, the second horticultural light source being configured to illuminate a test plant or crop according to a test lighting scenario; in the control zone, illuminating the control plant or crop with the first horticultural light source according to the control lighting scenario; in the test zone, illuminating the test plant or crop with the second horticultural light source according to the test lighting scenario in the test zone; and following a release of pollinators within the horticultural structure, determining whether the test lighting scenario impacts a pollination-related property of the test plant or crop with respect to a pollination-related property of the control plant or crop.

In some embodiments, defining the control zone includes creating the control zone, delimiting the limits of the control zone and/or adjusting the dimensions of the control zone.

In some embodiments, defining the test zone includes creating the test zone, delimiting the limits of the test zone and/or adjusting the dimensions of the test zone.

In some embodiments, the method further includes monitoring at least one of the control lighting scenario and the test lighting scenario to collect information on past, actual and/or forecasted lighting scenarios.

In some embodiments, the method includes comprising correlating the information on the past, actual and/or forecasted lighting scenarios with the pollination-related property of the test plant or crop and/or the pollination-related property of the control plant or crop.

In accordance with one aspect, there is provided a system for determining pollination illumination patterns within a horticultural structure. The system includes a first horticultural light source configured to illuminate a control plant or crop according to a control lighting scenario; a second horticultural light source configured to illuminate a test plant or crop according to a test lighting scenario; at least one sensor configured to monitor a pollination-related property of the test plant or crop and a pollination-related property of the control plant or crop; and at least one controller in data communication with the first horticultural light source, the second horticultural light source and said at least one sensor, said at least one controller being configured for: defining a control zone within the horticultural structure, the control zone being associated with a first horticultural light source; defining a test zone within the horticultural structure, the test zone being associated with a second horticultural light source; sending first illumination instructions to the first horticultural light source to irradiate the control plant or crop according to the control lighting scenario in the control zone; sending second illumination instructions to the second horticultural light source to irradiate the test plant or crop according to the test lighting scenario in the test zone; and following a release of pollinators within the horticultural structure, determining whether the test lighting scenario impacts a pollination-related property of the test plant or crop with respect to a pollination-related property of the control plant or crop.

In some embodiments, the system includes a first sensor associated with the control plant or crop to track the pollination-related property of the control plant or crops.

In some embodiments, the system includes a second sensor associated with the test plant or crop to track the pollination-related property of the test plant or crop.

In some embodiments, at least one of the first sensor and the second sensor is a camera or a photodetector.

In some embodiments, the first sensor and the second sensor are each configured to detect characteristics associated with flying insects.

In some embodiments, the characteristics are at least one of a species of the flying insects, a sex of the flying insects, a physiological state of the flying insects and a relative position of the flying insects with respect to the first horticultural light source and/or the second horticultural source.

In some embodiments, the first sensor and the second sensor are each configured to detect a presence or an absence of the flying insects.

In some embodiments, the system includes a user interface allowing an end user to enter a pattern discovery strategy as a programming language or a visual programming interface.

In some embodiments, each one of the control zone and the test zone includes one or more rows of plants or crops.

In some embodiments, the first horticultural source and the second horticultural source each include a dedicated controller.

In accordance with one aspect, there is provided a non-transitory computer readable storage medium having stored thereon computer executable instructions that, when executed by a processor, cause the processor to perform the methods herein disclosed, or at least one step of the methods.

The present techniques also generally relate to automated system and related method for enabling the execution or implementation of programmed light-driven strategies aimed at attracting or driving pollinators within a horticultural structure, in the context of controlled environment agriculture. Broadly described, the techniques that will be described allow retaining pollinators at a specific location within the environment of the horticultural structure, stimulating or assisting the pollination process at a specific location within the horticultural structural, and also repulsing flying insects from a specific location. The present techniques rely on a supplemented lighting scenario to guide the pollinators within the horticultural structure, while simultaneously implementing a lighting scenario having optical properties aimed at achieving photosynthetic growth of plants or crops.

In accordance with one aspect, there is provided a method for assisting pollination within a horticultural structure. The method includes defining a first zone within the horticultural structure, the first zone being associated with a first horticultural light source, the first horticultural light source being configured to illuminate a first group of plants or crops according to a lighting scenario, the lighting scenario having optical properties based on a targeted photosynthetic growth of the first group of plants or crops; defining a second zone within the horticultural structure, the second zone being associated with a second horticultural light source, the second horticultural light source being configured to illuminate a second group of plants or crops according to a supplemented lighting scenario, the supplemented lighting scenario having at least one expected pollination-enhancing feature; in the first zone, illuminating the first group of plants or crops with the first horticultural light source according to the lighting scenario; and in the second zone, illuminating the second group of plants or crops with the second horticultural light source according to the supplemented lighting scenario.

In some embodiments, the method further includes monitoring a distribution of pollinators within the first zone and the second zone to determine whether said at least one expected pollination-enhancing feature affects the distribution of pollinators within the second zone in comparison with the first zone.

In accordance with one aspect, there is provided a system for assisting pollination within a horticultural structure. The system includes a first horticultural light source configured to illuminate a first group of plants or crops according to a lighting scenario, the lighting scenario having optical properties based on a targeted photosynthetic growth of the first group of plants or crops; a second horticultural light source configured to illuminate a second group of plants or crops according to a supplemented lighting scenario, the supplemented lighting scenario having at least one expected pollination-enhancing feature; and at least one controller in data communication with the first horticultural light source, the second horticultural light source and said at least one sensor, said at least one controller being configured for: defining a first zone within the horticultural structure, the first zone being associated with the first horticultural light source; and defining a second zone within the horticultural structure, the second zone being associated with the second horticultural light source.

In some embodiments, the system further includes at least one sensor configured to monitor a distribution of pollinators within the first zone and the second zone, and said at least one controller is further configured for determining whether said at least one expected pollination-enhancing feature of the supplemented lighting scenario affects the distribution of the pollinators within the second zone in comparison with the first zone, based on the monitoring carried out by said at least one sensor.

In accordance with one aspect, there is provided a non-transitory computer readable storage medium having stored thereon computer executable instructions that, when executed by a processor, cause the processor to perform the methods herein disclosed, or at least one step of the methods.

Other features and advantages of the method and system described herein will be better understood upon a reading of preferred embodiments thereof with reference to the appended drawings. Although specific features described in the above summary and in the detailed description below may be described with respect to specific embodiments or aspects, it should be noted that these specific features can be combined with one another unless stated otherwise.

### BRIEF DESCRIPTION OF THE DRAWINGS

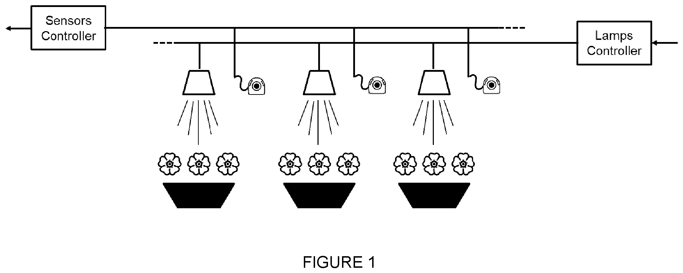

Figure 1 shows a system for determining pollination illumination patterns within a horticultural structure, in accordance with one embodiment.

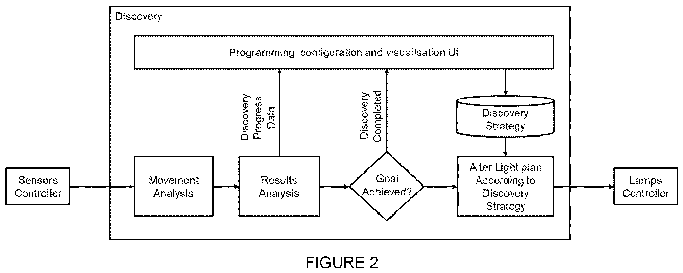

Figure 2 illustrates how features of the system depicted in Figure 1 cooperate with each other, in accordance with one embodiment.

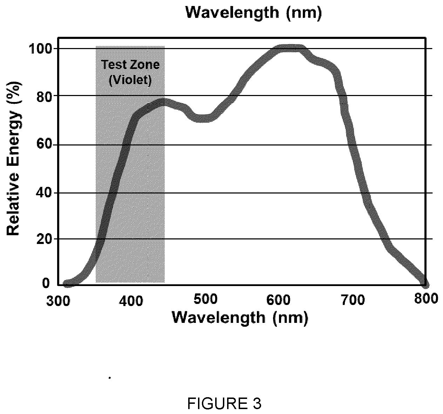

Figure 3 shows an illumination spectrum associated with a test lighting scenario that can be delivered in a test zone of a horticultural structure, in accordance with one embodiment.

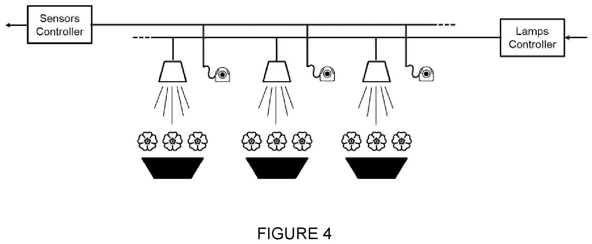

Figure 4 shows a system for determining pollination illumination patterns within a horticultural structure, in accordance with one embodiment.

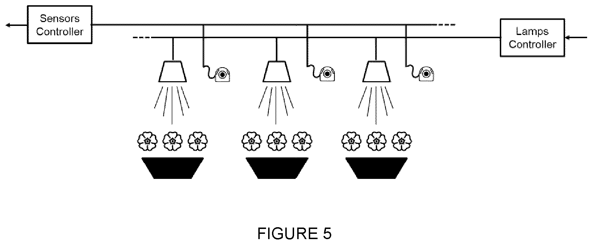

Figure 5 shows a system for determining pollination illumination patterns within a horticultural structure, in accordance with one embodiment.

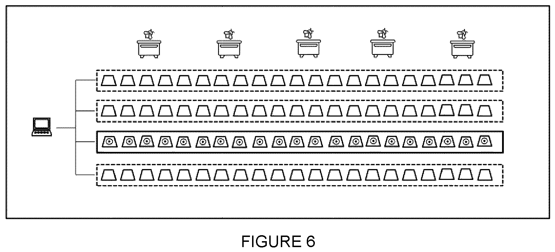

Figure 6 illustrates an implementation of the techniques for determining pollination illumination patterns within a horticultural structure.

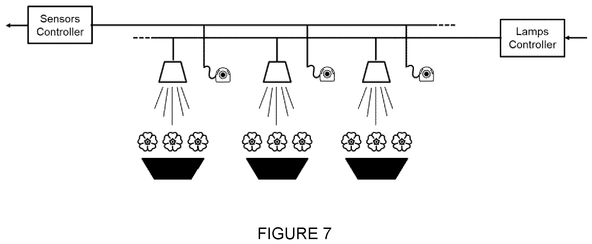

Figure 7 shows a system for assisting pollination within a horticultural structure, in accordance with one embodiment.

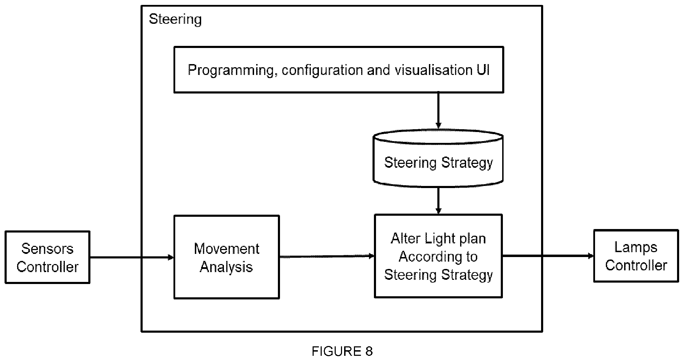

Figure 8 illustrates how features of the system depicted in Figure 7 cooperate with each other, in accordance with one embodiment.

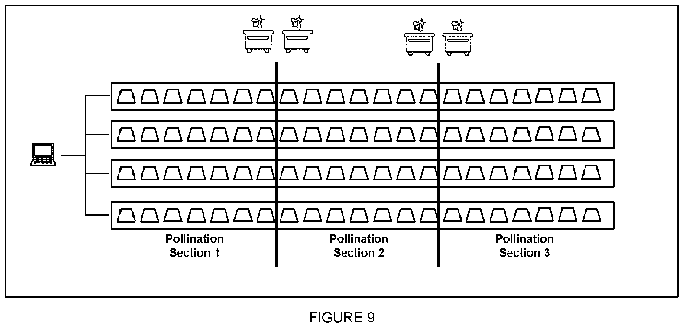

Figure 9 is an illustration of a horticultural structure being virtually separated into three virtual zones, in accordance with one embodiment.

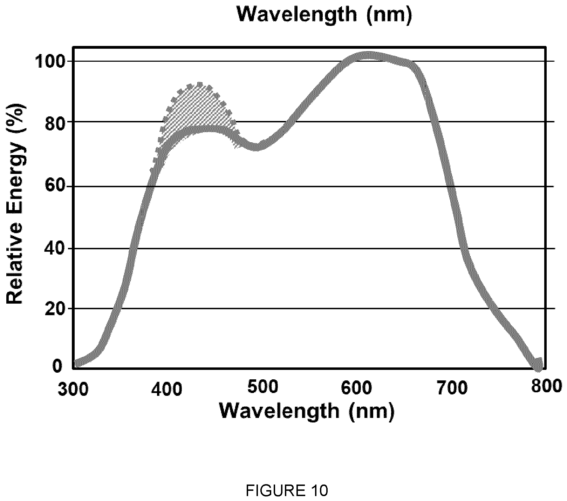

Figure 10 shows an illumination spectrum associated with a supplemented lighting scenario that can be delivered in a second zone of a horticultural structure, in accordance with one embodiment.

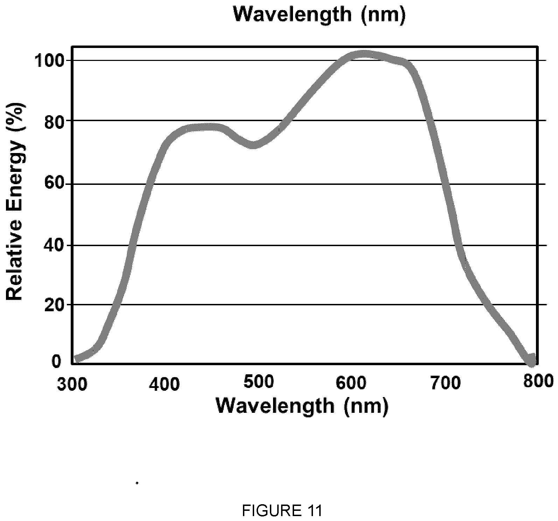

Figure 11 shows an illumination spectrum associated with a lighting scenario that can be delivered in a first zone of a horticultural structure, in accordance with one embodiment.
Figure 12 shows how a distribution of pollinators can be controlled in one of the three virtual zones illustrated in Figure 9.

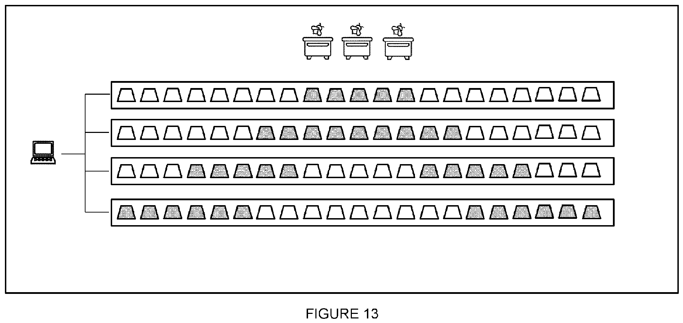

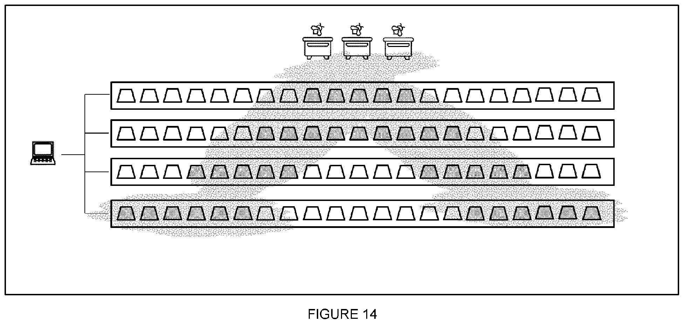

Figures 13 and 14 show how a corridor can be formed using lamps provided in a horticultural structure, in accordance with one embodiment.

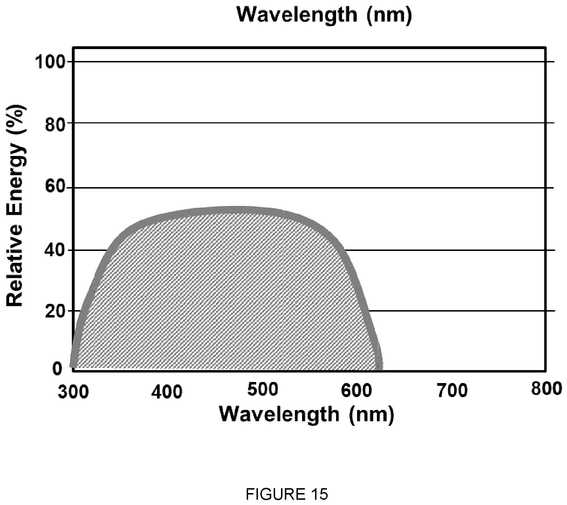

Figure 15 illustrates expected pollination-enhancing feature of a supplemented lighting scenario, in accordance with one embodiment.

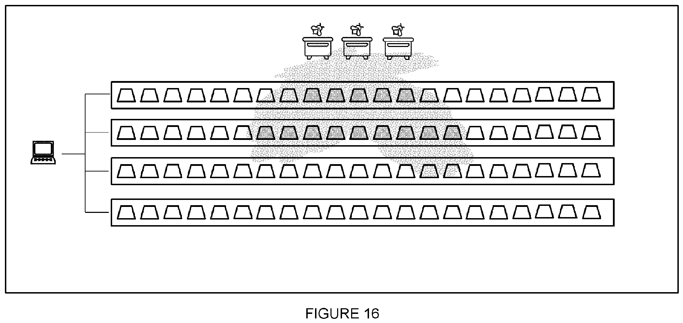

Figure 16 shows how pollinators can be guided back towards their hive, in accordance with one embodiment.

### DETAILED DESCRIPTION

In the present description, similar features in the drawings have been given similar reference numerals. To avoid cluttering certain figures, some elements may not have been indicated if they were already identified in a preceding figure. It should also be understood that the elements of the drawings are not necessarily depicted to scale, since emphasis is placed on clearly illustrating the elements and structures of the present embodiments. Furthermore, positional descriptors indicating the location and/or orientation of one element with respect to another element are used herein for ease and clarity of description. Unless otherwise indicated, these positional descriptors should be taken in the context of the figures and should not be considered limiting. More particularly, it will be understood that such spatially relative terms are intended to encompass different orientations in the use or operation of the present embodiments, in addition to the orientations exemplified in the figures.

The terms "a", "an" and "one" are defined herein to mean "at least one", that is, these terms do not exclude a plural number of items, unless stated otherwise.

Terms such as "substantially", "generally" and "about", that modify a value, condition or characteristic of a feature of an exemplary embodiment, should be understood to mean that the value, condition or characteristic is defined within tolerances that are acceptable for the proper operation of this exemplary embodiment for its intended application.

Unless stated otherwise, the terms "connected" and "coupled", and derivatives and variants thereof, refer herein to any structural or functional connection or coupling, either direct or indirect, between two or more elements. For example, the connection or coupling between the elements may be acoustical, mechanical, optical, electrical, thermal, logical, or any combinations thereof.

The terms "match", "matching" and "matched" are intended to refer herein to a condition in which two elements are either the same or within some predetermined tolerance of each other. That is, these terms are meant to encompass not only "exactly" or "identically" matching the two elements but also "substantially", "approximately" or "subjectively" matching the two elements, as well as providing a higher or best match among a plurality of matching possibilities.

In the present description, the expression "based on" is intended to mean "based at least partly on", that is, this expression can mean "based solely on" or "based partially on", and so should not be interpreted in a limited manner. More particularly, the expression "based on" could also be understood as meaning "depending on", "representative of", "indicative of", "associated with" or similar expressions.

It should be noted that, in the context of the current disclosure, the expression "plant(s) or crop(s)" may encompass a broad variety of multicellular organisms, including photosynthetic eukaryotes. Non limitative examples of plant(s) or crop(s) are seedlings, ornamental crops, ornamental plants, plugs, liners, fruits, small fruits, vegetables, leafy greens, herbs, young plants, high-value crops, and many others. In the context of the current disclosure, the plants or crops can be pollinated. The plants or crops may be produced for human food, non-human food or non-food applications. The growing process of the plants or crops generally includes a plurality of subsequent plant growth stages, such as, for example, seed germination (or "sprout"), seedling, vegetative, bud stage (or "budding"), flowering and ripening. It should be understood that, in the present description, the plants or crops can be at any one of the plant growth stages or at a transition between any two subsequent growth stages.

The expression "horticultural light", synonyms and derivatives thereof will be used throughout the present disclosure, and refers to the use of optical techniques, systems, and methods for assisting, maintaining, stimulating and/or optimizing plants or crops growth. The horticultural light may irradiate or illuminate the plants or crops during any one of the plant growth stages. The horticultural light, which is the light generated by the horticultural lighting apparatus, may be produced or generated using an artificial light source or similar devices, apparatuses, and systems. Non-limiting examples of artificial light sources include incandescent light sources, fluorescent light sources, high-intensity discharge (HID) light sources such as mercury vapor, metal halide (MH), high-pressure sodium (HPS) and low-pressure sodium (LPS) light sources, solid-state light sources including LED light sources, and laser sources. In the context of the current disclosure, the artificial light sources have adjustable optical properties. The horticultural light is associated with an illumination spectrum or profile. In some implementations, the horticultural light produced by the horticultural lighting apparatus have a profile substantially similar to light reaching the crop or plant. The expression "illumination spectrum" is used to broadly refer to the spectral power distribution of an illumination. The illumination spectrum can represent the distribution of power radiated per unit area and per unit wavelength or frequency over a spectral region of the electromagnetic spectrum. It should be noted that using horticultural light may be used to irradiate or illuminate plants or crops growing in a horticultural structure providing regulated climatic conditions to the plants or crops. Nonlimitative examples of horticultural structures include greenhouse, glasshouse and hothouse.

In the present description, the terms "light" and "optical", and variants and derivatives thereof, are used to refer to radiation in any appropriate region of the electromagnetic spectrum. The terms "light" and "optical" are therefore not limited to visible light, but can also include, without being limited to, the infrared and ultraviolet regions. For example, in some implementations, the present techniques can be used with electromagnetic signals having wavelengths ranging from about 250 nm to about 2500 nm. However, this range is provided for illustrative purposes only and some implementations of the present techniques may operate outside this range. Also, the skilled person will appreciate that the definition of the ultraviolet, visible and infrared ranges in terms of spectral ranges, as well as the dividing lines between them, can vary depending on the technical field or the definitions under consideration, and are not meant to limit the scope of applications of the present techniques.

The expressions "natural light" or "natural light conditions" generally refer to light having spectral characteristics corresponding or similar to those of sunlight, moonlight or starlight. The spectral profile of natural light, particularly sunlight, varies as a function of geographic location, time of day, time of year, weather, cloud coverage, and several other factors. Several standards are known in the art to provide a spectral reference for natural light. For example, the Commission internationale de l'eclairage (CIE) has established the D series of well-defined daylight standard illuminants representing natural light under different conditions. One well-known standard is CIE Standard Illuminant D65, which is a daylight illuminant that intends to represent the average midday light in Western or Northern Europe. Other examples of CIE Standard Illuminants for daylight include the D50, D55, and D75 standard illuminants. Sunlight, which refers to the total spectrum of electromagnetic radiation emitted by the Sun and reaching the Earth, has a broad spectral range including ultraviolet radiation, visible light, and infrared radiation. Accordingly, standard illuminants extend within the solar radiation spectrum. For example, Standard Illuminant D65 extends from 300 nm to 830 nm. Non-limiting examples of natural light sources include sunlight, moonlight, starlight, twilight, lightning, and firelight.

In the present description, the term "solid-state light emitter" refers to any light-emitting device that converts electrical energy into electromagnetic radiation through the recombination of electronic carriers (i.e., electrons and holes) in a light emitting layer or region. The emitting layer or region can include, but is not limited to, silicon, silicon carbide, gallium nitride and/or other semiconductor materials, and may or may not include a substrate such as sapphire, silicon, silicon carbide and/or other microelectronic substrates. The solid-state light emitters can include both inorganic and organic light emitters, many of which are known to the skilled person and need not be described in detail herein. Non-limiting examples of types of solid-state light emitters include semiconductor light-emitting diodes (LEDs), semiconductor laser diodes, vertical cavity surface emitting lasers (VCSELs), other semiconductor light emitting devices or lamps, organic light-emitting diodes (OLEDs), and polymer light-emitting diode (PLEDs).

The expression "lighting scenario" is understood to refer to the generation of light, such as for illuminating purposes, according to predetermined optical characteristics (e.g., spectral content, intensity, polarization) that may, in some instances, change, vary or evolve over time during a given time period. In other instances, the predetermined optical characteristics may remain constant, unchanged or not significantly evolve over time, during a given duration or period, (e.g., during a cycle or a portion of the lighting scenario). The embodiments wherein the optical characteristics change, vary or evolve over time may sometimes be referred to as "dynamic lighting scenario". The embodiments wherein the optical characteristics remain constant, unchanged or not significantly evolve may sometimes be referred to as "static lighting scenario". The expression "lighting scenario", which will be used throughout the present description, encompasses both variants, i.e., a dynamic lighting scenario and a static lighting scenario, as well as any combinations thereof. The optical characteristics of the generated light may correspond to or emulate those of natural lighting conditions. The natural light may emulate or be inspired from the actual light conditions experienced at a specific geographical location, date and time. It is appreciated that devising lighting scenarios that combine natural light conditions corresponding to different geographical locations is possible in some applications (e.g., a scenario could be build using sunrise, midday and sunset conditions corresponding to three distinct locations on Earth, at the same or different dates). In other embodiments, however, the natural light conditions may be different from real life conditions on Earth. By way of example, the spectrum of natural light generated according to the method described herein may differ from an actual spectral content of sunlight due to the absence of spectral components which are undesired or unnecessary in a given application context, or conversely by the enhancement or addition of wavelengths then are considered advantageous or required. The present techniques may rely on the use of solid-state light emitters. The solid-state light emitters can be driven to produce the lighting scenario using sets of control parameters. It should be noted that a plurality of lighting scenarios may be combined to collectively determine a "recipe" or a "recipe bundle". The recipe or the recipe bundle refers to a sequence of lighting scenarios.

In some embodiments, the lighting scenario may emulate lighting conditions over the course of a day, from dawn to dusk, or over a portion of a day. Indeed, the spectral contents of light reaching a particular location on earth from the Sun is not constant as the day progress. In some instances, it can be customary to characterize natural light according to its Correlated Color Temperature (CCT) value, expressed in Kelvin (K). By convention, the CCT is defined by the CIE as "the temperature of the Planckian radiator whose perceived color most closely resembles that of a given stimulus at the same brightness and under specified viewing conditions" (CIE/IEC 17.4:1987, International Lighting Vocabulary). Lower CCT values correspond to "warmer" light. Hence, a day with a clear blue sky can begin at dawn with light in a warm CCT spectrum range, such as between 1500K and 3000K, then progress to about 5000K to 7500K at mid-day and return to the 1500K to 3000K range towards dusk. In horticultural or agricultural applications, the light conditions in a region of the world from which a cultivated produce originates or where this produce is known to thrive can be emulated (e.g., growing tomatoes using light conditions from a sunny day in June in Tuscany). In other examples, the lighting conditions may be adapted in view of observations or discoveries regarding optimal or enhanced lighting conditions for growing a given agricultural output, such as for example to follow the McCree Curve, which represents the average photosynthetic response of plants to light energy.

In the context of the current disclosure, the expression "pollination-related property" refers to at least one of: pollinators' concentration within a specific area, frequency and speed of movements, frequency and speed of takeoff and landings, spatial distribution, general proximity with flowers, relative intensity of the buzz sound, and many other similar characteristics.

### Natural Pollination

The present techniques broadly relate to industrial greenhouse and controlled environment agriculture, *i.e.,* horticultural structures relying on the use of pollinators in the context of the pollination process of plants or crops.

In crop production, the pollination process helps the plants producing their fruits. While some plants are self-pollinating, *i.e.,* have the ability to transfer pollen from a male anther to a female stigma without any external assistance or support, many plants rely on pollinators, such as insects, during their pollination process, so that their flowers can mature.

In external environments, natural processes, which may include wind, bees, wasps, birds, butterflies, moths, flies, and many others, can play the role of pollinators. However, in controlled environment agriculture, the options are typically reduced and often limited. Bumblebees are commonly used pollinators in controlled environment agriculture. It has been found that using bumblebees to pollinate plants inside a greenhouse is one way to improve crop quality, increase yield, and reduce labor.

In controlled environment agriculture, bumblebees are typically delivered to the growers in hives, temporarily positioned in a cardboard box. The hives are positioned near or around the plants or crops, and, once the box containing the bumblebees is adapted to the ambient conditions of the horticultural structure, the bumblebees are released. The bumblebees are then free to fly within the horticultural structure to retrieve or collect pollen as food, while carrying some pollen with them from plants to plants. Of note, the hive door is typically open at a moment corresponding to a natural or an artificial sunrise, or shortly after, when flowers are open or about to open. The hive door is later closed at a moment corresponding to a natural or an artificial sunset.

The conditions within the horticultural structure should be optimal or at least be optimized for the bumblebees, so that they can navigate in a relatively effortless manner. For example, the flowers should be relatively open and relatively easy to detect. The path between the flowers, plants or crops should be such that the bumblebees are able to fly out and back of the hive, without losing their way.

It has been found that bumblebees can see pr process wavelengths from approximately 300 nm to approximately 650 nm, which mean that they cannot see the color red, but that they can see in the ultraviolet portion of the electromagnetic spectrum (contrary to the humans). Bumblebees can also easily distinguish between dark colors and light colors, which facilitates detecting edges of leaves or flowers, or the contour of objects present in their environment.

Vision is the mechanism through which bumblebees locate flowers on which to feed. Bumblebees' vision is relatively sensitive to the ultraviolet portion of the electromagnetic spectrum (Kevan *et al*.)*.* Many flowers have distinctive ultraviolet color patterns that are invisible to the human eyes but are incredibly eye-catching to bumblebees. These ultraviolet patterns often outline landing zones or regions for the bumblebees, indicating which part of the plant contains nectar and pollen (Whitney *et al.,* 2013). Color-related properties, such as iridescence and contrasts against the background environment, have been demonstrated to play a significant role in flower search time, flight behavior, landing, and pollination efficiency (Lunau, 2016, and Spaethe *et al.,* 2001).

It should be noted that direct light and indirect light both have a significant impact on an insect's vision and, consequently, estimating pollination productivity is still a challenge. For example, and without being limitative, refracted light in greenhouses can confuse bumblebees or honeybees.

Some of the techniques herein described allow estimating or characterizing the impacts of ambient conditions, which include direct light and indirect light, on the pollinators' behavior in controlled environment agriculture. Based on this estimation or characterization, some of the present techniques allow adjusting or supplementing the horticultural light irradiating the plants or crops within the horticultural structure. Some of the techniques herein described allow using knowledge about the impacts of ambient conditions, which include direct light and indirect light, on the pollinators' behavior in controlled environment agriculture. Based on this knowledge, the present techniques allow adjusting, guiding, assisting displacement of pollinators within a horticultural structure, by supplementing or adjusting the horticultural light irradiating the plants or crops within the horticultural structure, with the ultimate objective of promoting or facilitating a targeted photosynthetic growth of a group of plants or crops using at least one expected pollination-enhancing feature of light, as it will be described in greater detail hereinbelow.

It is also known that vision of bees and bumblebees rely on motion parallax (Lehrer, 1998). As an insect flies over a field of flowers, the flower that is closer to the insect with respect to its background appears to move faster, thereby creating a relative motion between the flower and the background. In natural environment, the flowers are nearly always moving, typically due to wind, and can thus be easily detected by the insects. However, in a controlled environment, such as a greenhouse, there is typically no wind, which complicate the detection of flowers by the insects.

Reflection of light on plant leaves is known to influence their attractivity to the insects. The literature on this topic suggests that it is the color contrast of a flower with its background that influences bees' preferences (Whitney *et al*.). However, the variables affecting the visual perception of bees or bumblebees are nearly countless: type of crops and flowers, physical environment, insects' innate or acquired behaviors, insect variety, growth climate, and many other factors.

Ensuring optimal insect productivity becomes even more complex when artificial lighting is involved, for instance with supplemental lighting, also known as assimilation lighting, which is nearly always present in greenhouses or in controlled environment agriculture. As modern LED based technologies tend to evolve toward variable intensity and modulable dynamic spectrums, the overall complexity of evaluating the pollination efficiency increases. In some cases, artificial lighting can have lethal effects on insects' population (Blacquiere *et al.,* 2007).

Colors correspond to wavelengths that are either reflected or transmitted, i.e., an object absorbs some wavelengths and reflects others. Past experiments in the field of optimizing the lighting conditions of a horticultural structure were limited by the characteristics of available light sources (incandescent, fluorescent, high-pressure sodium, and many others). In order to experiment with a wider spectrum of colors, an experimenter had to use matter to reflect and absorb wavelengths, to generate target perceived colors.

The relatively young invention of the LED technology and recent breakthroughs in the ability to manufacture LEDs of various colors have made it possible to modulate the energy of individual wavelengths to create very specific light spectrums. The present techniques are implemented in this paradigm. More specifically, some of the present techniques allow discovering and experimenting with insects' behavioral response to light. Some of the present techniques allow exploiting, guiding, influencing and/or affecting insects behavioral response to light in order to achieve a targeted photosynthetic growth of a group of plants or crops.

The availability of connected growth lighting systems (see for example PCT/CA/2016/050076 and PCT/CA2019/051633) that are individually capable of variable intensity, modulable, static, and dynamic spectrums represents new opportunities for the growers. Indeed, it becomes possible to use the light generated by the artificial lighting systems to alter bees' behavior in order to guide and optimize their pollination work. Dynamic grow lights can be globally or individually controlled to provide light patterns aiming at inhibiting undesirable insects' behaviors, or to stimulate desirable behaviors, while simultaneously providing a light spectrum capable of generating photosynthetic growth. For instance, growth lighting systems can be modulated to attract bees toward specific locations, specific plant clusters. This can be used for ensuring uniform pollination across the crop, to achieve targeted pollination or to keep bees away from specific sections of the grow area. Light could also be used to facilitate bees' sunset fly back at the hive, i.e., to indicate that the pollination process is coming to an end. Other potential applications of locally controlled lighting include, but are not limited to facilitation of pollination by optimizing discovery, approach, landing and controlling bees' retention time at given location.

Any single controlled growth environment, with its crop genetic particularities, its lightning characteristics, its climate, to only name a few, has its own unique characteristics and challenges. What was observed to work in a particular growth environment, with given bee's genetics, may work or may not work within another environment.

For that reason, tools and methodologies enabling the discovery, execution and/or application of light patterns and strategies are required not only in a laboratory environment but in the production environment as well. The present techniques allow harnessing the potential of using light to optimize the desired behaviors of insects.

### Determining Pollination Illumination Patterns

The present description relates to techniques, including an automated system and corresponding methods, enabling the experimental discovery and classification of static and dynamic light patterns capable of attracting flying insects or pollinators towards a specific or determined location within the controlled agriculture environment, retaining insects at a specific or determined location within the controlled environment, stimulating their target activity at a specific or determined location and repulsing flying insects from a specific or determined location.

In accordance with one aspect, there is provided a system for determining pollination illumination patterns within a horticultural structure. The system includes a first horticultural light source configured to illuminate a control plant or crop according to a control lighting scenario and a second horticultural light source configured to illuminate a test plant or crop according to a test lighting scenario. Of note, the first horticultural light source and the second horticultural light source may each be embodied by a plurality of lamps (*i.e.,* a pool of lamps). The system also includes at least one sensor configured to monitor a pollination-related property of the test plant or crop and a pollination-related property of the control plant or crop. In some embodiments, the control plant or crop may be associated with a first sensor and the test plant or crop may be associated with a second sensor. In some embodiments, the sensor may be embodied by a camera, a photodetector, or any other devices that can be used to track a pollination-related property of the plants or crops. The system also includes at least one controller in data communication with the first horticultural light source, the second horticultural light source and the sensor(s). The controller is configured for performing a series of steps, which may include, for example, defining a control zone within the horticultural structure, the control zone being associated with the first horticultural light source; defining a test zone within the horticultural structure, the test zone being associated with the second horticultural light source; sending first illumination instructions to the first horticultural light source to irradiate the control plant or crop according to the control lighting scenario in the control zone; sending second illumination instructions to the second horticultural light source to irradiate the test plant or crop according to the test lighting scenario in the test zone; and following a release of pollinators within the horticultural structure, determining whether the test lighting scenario impacts a pollination-related property of the test plant or crop with respect to a pollination-related property of the control plant or crop.

In some embodiments, the system includes at least two lamp fixtures adapted to generate variable spectrum output and/or have variable intensity capabilities. In these embodiments, the system also includes one or more sensors adapted to detect the presence and activity of flying insects, and their relative location in relation with the lamp fixtures (as illustrated in US 2021/0360204). Optionally, the system may include one or more sensors adapted to detect characteristics associated with the flying insects, such as species, sex, physiological state, and other characteristics (see for example US Patent 10,178,856 B2). In these embodiments, the system includes a first controller capable of controlling individual fixtures' visible and non-visible light spectrum output and intensity and a second controller capable of receiving sensor information. Of note, the first and second controllers could be implemented in a single controller or could otherwise be independent one from another. In these embodiments, the lamps fixtures, sensors and controllers are in data communication, using either wired connections or wireless connections. It should be noted that the expression "data communication" may refer to any types of direct connection and/or indirect connection. For example, the communication between the elements may be achieved through direct communication such as a wired connection or via a network allowing data communication between devices or components of a network capable of receiving and/or sending data, which may include, to name a few, publicly accessible networks of linked networks, possibly operated by various distinct parties, such as the Internet, private networks (PN), personal area networks (PAN), local area networks (LAN), wide area networks (WAN), cable networks, satellite networks, cellular telephone networks and the like, or any combinations thereof.

In these embodiments, the system also includes a user interface allowing an end user to enter a pattern discovery strategy, for instance, in the form of a programming language or a visual programming interface. The system can be programmed to use techniques such as logic programming, trial and error, statistical correlation, machine learning, deep learning and any other learning strategy to perform the discovery process. In these embodiments, the system also includes a processor capable of using the sensor data to measure insects' response to the light patterns with regards to insects' attraction, retention, stimulation and repulsion, alter and execute light patterns on lamps fixtures.

The embodiments of the horticultural structure illustrated in the Figures include at least two zones, namely the control zone and the test zone. Each zone may include one or more rows of plants or crops. Each zone is designed or configured to accommodate the plants or crops during one or more plant growth stages (or portions thereof).

In some embodiments, the first and second horticultural light sources may be embodied by a lamp similar to the lighting system described in WO 2016119063, the content of which is incorporated herein by reference.

The controller(s) are configured to send illumination instructions to one of the horticultural light sources, such that the corresponding horticultural light source is driven according to a lighting scenario to meet the lighting conditions of the plants or crops. In some embodiments, each horticultural light source includes a dedicated controller, and each dedicated controller is operatively connected to a user interface. The user interface may be configured to select one of horticultural light source, receive information about the illumination sequence or the lighting scenario being provided by the selected horticultural light source during portion(s) of a lighting scenario and/or send illumination instructions to the selected horticultural light source. As such, the user interface may be in data communication with each dedicated controller of the horticultural light sources through a corresponding communication channel. Of note, the dedicated controller of each horticultural light source may communicate with the user interface over a network. The term "network", synonyms and derivatives thereof are used to refer to any classes of network, which includes publicly accessible networks of linked networks, possibly operated by various distinct parties, such as the Internet, private networks, personal area networks, local area networks, wide area networks, cable networks, satellite networks, cellular telephone networks or any combinations thereof.

In some embodiments, the user interface may be a graphical user interface. In some embodiments, the user interface may be a conversational interface, which may rely on text-activated commands and/or voice-activated commands. The user interface is operatively connected to at least one of the horticultural lighting sources through at least one controller to allow a user to interact with the horticultural lighting sources. In some embodiments, the user interface may be displayed on a display or a screen. In some embodiments, the graphical user interface may be part of a web-based application that may be accessed and displayed using a computing device connected to the Internet or any types of networks. The user interface may also be configured to illustrate the paths followed by the pollinators, which may help identifying optimal lighting conditions for the plants or crops.

In some embodiments, the system may include a memory, or may include or be connected to a database (*e.g.,* through the controller) to store past lighting scenarios or illumination conditions of the plants or crops, calibration data, other relevant data, as well as the impacts of these parameters on the pollination-related properties of the test plant or crop with respect to the control plant or crop. In some embodiments, the lighting scenarios may be provided in a dataset including a plurality of sets of control parameters for the horticultural light sources. Each set of control parameters may be associated to an illumination state of the horticultural light source, and associated with the impact on the pollination process. The dataset may be stored as a relational database and may have a database format commonly used in the art, such as, for example and without being limitative, CSV/SCSV, SQL, NoSQL, Graph DB, Object Oriented DB, Tabular DB, Large Language Model (LLM), or the like. The dataset may comprise textual information, numeral information, time information, date information, image information, and any combinations thereof. In the context of controlling horticultural light, the control parameters may include the different driving parameters of the horticultural light.

The system having been described may be implemented in computer programs executed on programmable computers. A programmable computer generally includes at least a processor and a data storage system that may include volatile and non-volatile memory and/or storage elements. The programmable computer may be a programmable logic unit, a mainframe computer, server, and personal computer, cloud-based program or system, laptop, personal data assistance, cellular telephone, smartphone, wearable device, tablet device, virtual reality devices, smart display devices, set-top box, video game console, portable video game devices, or virtual reality device. In some embodiments, the systems and methods may be provided as a plug-in. In some embodiments, one or more components of the system having been described may be provided as a plug-in. The expression "plug-in" herein refers to a software component adding a predetermined feature or functionality to the system. Providing the different modules as plug-ins may be associated with some benefits, such as, for example and without being limitative, adaptability, modularity and flexibility.

Of note, the computer programs may be implemented in a high-level procedural or object-oriented programming and/or scripting language to communicate with a computer system. The programs could alternatively be implemented in assembly or machine language, if desired. In these implementations, the language may be a compiled or interpreted language. The computer programs are generally stored on a storage media or a device readable by a general or special purpose programmable computer for configuring and operating the computer when the storage media or device is read by the computer to perform the procedures described herein. In some embodiments, the systems may be embedded within an operating system running on the programmable computer.

In accordance with one aspect, there is provided a method for determining pollination illumination patterns within a horticultural structure.

The method includes a step of defining a control zone within the horticultural structure, the control zone being associated with a first horticultural light source. The first horticultural light source is configured to illuminate a control plant or crop according to a control lighting scenario.

The method also includes a step of defining a test zone within the horticultural structure, the test zone being associated with a second horticultural light source. The second horticultural light source is configured to illuminate a test plant or crop according to a test lighting scenario.

The method also includes a step of illuminating the control plant or crop with the first horticultural light source according to the control lighting scenario in the control zone. Similarly, the method also includes a step of illuminating the test plant or crop with the second horticultural light source according to the test lighting scenario in the test zone.

The method also includes a step of determining whether the test lighting scenario impacts a pollination-related property of the test plant or crop with respect to a pollination-related property of the control plant or crop, following a release of pollinators within the horticultural structure.

The method also includes a step of monitoring information representative of one or more pollination-related properties of the plants or crops during the control lighting scenario and the test lighting scenario, in order to identify which optical properties optimizes or improves the pollination process within the horticultural structure.

In some embodiments, defining the control zone and the test zone may include creating the zones, delimiting their limits, adjusting their dimensions, and the like. These steps may be carried out through the user interface. The zones may be determined using computer programs and/or vocal recognition-based approaches (*e.g.,* vocal commands). For example, the user interface may be configured to display a representation of the horticultural structure and the horticultural light sources, interact with the representation of the horticultural structure (and components). The user interface may further be configured to create, delete, duplicate, replicate, edit and/or reorganize the zones, based on the plants or crops present in the horticultural structure and their needs.

In some embodiments, the method may include monitoring or tracking the lighting scenarios or recipes according to which the horticultural lighting sources are driven to provide information on the past, actual and/or forecasted lighting scenarios. This information may be correlated with one or more pollination-related properties of the plants or crops. In some embodiments, the method may include editing or adjusting the lighting scenario based on the correlation between the illumination conditions during the lighting scenarios (*i.e.,* control lighting scenario and test lighting scenario) and their impacts on the pollination-related properties.

In accordance with another aspect of the present description, there is provided a non-transitory computer readable storage medium having stored thereon computer executable instructions that, when executed by a processor, cause the processor to perform the methods that have been previously described. The non-transitory computer storage medium can be integrated to the systems or assemblies that have been described in the present description. The non-transitory computer storage medium could otherwise be operatively connected with the systems or assemblies. In the present description, the terms "computer readable storage medium" and "computer readable memory" are intended to refer to a non-transitory and tangible computer product that can store and communicate executable instructions for the implementation of various steps of the method disclosed herein. The computer readable memory can be any computer data storage device or assembly of such devices, including random-access memory (RAM), dynamic RAM, read-only memory (ROM), magnetic storage devices such as hard disk drives, solid state drives, floppy disks and magnetic tape, optical storage devices such as compact discs (CDs or CDROMs), digital video discs (DVD) and Blu-Ray^{™} discs; flash drive memory, and/or other non-transitory memory technologies. A plurality of such storage devices may be provided, as can be understood by those skilled in the art. The computer readable memory may be associated with, coupled to, or included in a computer or processor configured to execute instructions contained in a computer program stored in the computer readable memory and relating to various functions associated with the computer.

### Examples of Determining Pollination Illumination Patterns

Now that some embodiments of the technology have been described, some nonlimitative examples illustrating potential implementations of the techniques for determining pollination illumination patterns will be presented. It should be noted that these examples serve an illustrative purpose only and should therefore not be considered limitative.

According to a first example, a grower may want to identify an efficient color pattern capable of attracting and retaining bumblebees in the context of tomato culture. The literature on this topic suggests that the color contrast of a flower with its background greatly influences bees' preferences (Whitney *et al*.). The grower has made the empirical observation that violet light appears to enhance the color contrast of tomato flowers. Based on that information, the grower can elaborate a discovery strategy including determining a baseline spectrum to illuminate the plants or crops so that they achieve the desired photosynthetic growth, and then validating the approach. For example, the validation methodology may include testing the impact of 5% increases in energy between in a region of the electromagnetic spectrum extending between 350 nm to 450 nm, from the 40% energy generated by the baseline spectrum up to 100%. One possible outcome of this validation methodology is the percentage of bumblebee's mobility toward lamps with altered spectrum. The discovery phase may include testing numerous combinations of violet treatment durations and intensities and collecting data about bumblebee's mobility and retention under all illumination and treatment durations combinations. For example, the experimental environment may include three lamps capable of modulable spectrum and intensity, installed over three tomato plant clusters, with three camera-based sensors deployed in a manner allowing efficient detection of bumblebee's presence relative to the tomato plant clusters. The lamps and plants can be arranged side by side in a linear manner. The experimental environment is then populated with bumblebees. The system user interface is used by the grower to program computer instructions and configure the details of the execution of the discovery methodology. As the experimentation goes through iterations, the effectiveness of the influence of specific lighting and duration combinations can be automatically assessed using data obtained from the sensors. This data is saved for further review and can also be used to alter the direction of the discovery strategy. For instance, the strategy could be programmed to prioritize tests based on the most promising patterns and durations. The system allows the grower to validate hypotheses involving many samples, experiments that would be otherwise too tedious and, in some cases, unrealistic to perform manually.

According to a second example, a grower has been successfully using bumblebees to facilitate pollination across its production growth environment, and a new dynamic light recipe replicating natural sunlight daily circadian cycle, aimed at photosynthetic growth, has been made available. This new recipe however has a negative impact on crop pollination. Scouting, data collection and analysis has led the grower to observe that bees are significantly less active during the occurrence of certain illumination light spectrum patterns at specific times of the day. Following this observation, the grower identifies the undesirable light patterns negatively influencing bees' behaviors, experiments various adjustments that could be applied to the light spectrum and/or intensity of the baseline dynamic natural sunlight recipe and, to accelerate discovery, multiple light pattern candidates will be validated in parallel, in individual virtual test areas. The plan is then executed during normal production cultivation cycles. Large industrial greenhouses include multiple cultivation rows. For economic and technical reasons, only one row is equipped with sensors capable of capturing data about pollinators location and activity. In order to implement the discovery strategy, the sensing enabled row is virtually divided into seven sub-sections. Six of the seven sub-sections will be used to validate light patterns candidates or alternatives. The other section will be used for control and execute the baseline recipe. Four days will be allocated for validating a light pattern candidate. It is anticipated that twenty days of the growth cycle will be available for validation, resulting in the possibility to test 30 light pattern candidates during a single growth cycle. The discovery plan is implemented in two parallel channels. In the first channel, the grower will look at bees' activity patterns and use inferential statistics to make a model of relationships between bees' activity in relation to specific light sources used in the test and spectral characteristics of said light source. In the second channel, the grower will validate the light patterns candidates within the six virtual sub-sections of the sensor-enabled rows. Sensor data collected in these sub-sections is also absorbed by the first thread. The system user interface is used by the grower to program computer instructions and configure the details of the execution of the discovery methodology.

This experimental approach made possible by the present techniques makes it possible to perform large numbers of observations, correlation and experimentations that would otherwise be almost impossible to do manually. Additionally, in this case, the discovery methodology is entirely automated and seamlessly integrated with the production cycle.

### Assisting Pollination

The present description also relates to techniques, including an automated system and corresponding methods, for enabling the execution or implementation of programmed light-driven strategies aimed at attracting pollinators at specific or determined locations within a horticultural structure, in the context of controlled environment agriculture. The techniques that will be described allow retaining pollinators at a specific or determined location within the environment of the horticultural structure, stimulating or assisting the pollination process at a specific or determined location within the horticultural structural, and repulsing flying insects from a specific or determined location. The techniques that will be described rely on a supplemented lighting scenario to guide the pollinators within the horticultural structure, while simultaneously implementing a lighting scenario having optical properties aimed at achieving photosynthetic growth of plants or crops.

In accordance with one aspect, there is provided a system for assisting pollination within a horticultural structure. The system includes a first horticultural light source configured to illuminate a first group of plants or crops according to a lighting scenario and a second horticultural light source configured to illuminate a second group of plants or crops according to a supplemented lighting scenario. The lighting scenario has optical properties based on a targeted photosynthetic growth of the first group of plants or crops. The supplemented lighting scenario has at least one expected pollination-enhancing feature and allows implementing the lighting scenario having optical properties based on the targeted photosynthetic growth of the plants or crops. As such, the supplemented lighting scenario is a combination of a lighting scenario allowing the photosynthetic growth of the plants and crops, while allowing for precise intervention(s) during its implementation to guide the pollinators in a way that will be beneficial for the pollination process of the plants or crops. Of note, the first horticultural light source and the second horticultural light source may each be embodied by a plurality of lamps (*i.e.,* a pool of lamps). In some embodiments, the system may also include at least one sensor configured to monitor a distribution of pollinators within the first zone and the second zone. In some embodiments, the first group of plants or crops may be associated with a first sensor (or group of sensors) and the second group of plants or crops may be associated with a second sensor (or group of sensors). In some embodiments, the sensor may be embodied by a camera, a photodetector, or any other devices that can be used to track or monitor a distribution of pollinators within the horticultural structure. The system also includes at least one controller in data communication with the first horticultural light source, the second horticultural light source and the sensor(s). The controller is configured for performing a series of steps, which may include, for example, and without being limitative, defining a first zone within the horticultural structure, the first zone being associated with the first horticultural light source; defining a second zone within the horticultural structure, the second zone being associated with the second horticultural light source; and determining whether said at least one expected pollination-enhancing feature of the supplemented lighting scenario affects the distribution of the pollinators within the second zone in comparison with the first zone, based on the monitoring carried out by said at least one sensor.

In some embodiments, the system includes at least two lamp fixtures adapted to generate variable spectrum output and/or have variable intensity capabilities. In these embodiments, the system also includes one or more sensors capable of detecting the presence and activity of flying insects, and their relative location in relation with the lamp fixtures, including the first horticultural light source and the second horticultural light source (see for example US 2021/0360204). In some embodiments, the sensors are configured to detect characteristics associated with the pollinators or other flying insects, such as species, sex, physiological state, and other characteristics (see for example US Patent 10,178,856). The system according to these embodiments may also include a controller configured to control individual fixtures spectrum output (*i.e.,* the lighting scenario and/or lighting scenario according to which the first and/or second horticultural light source(s) illuminate the plants or crops). In some embodiments, the controller is configured to receive sensor information, *i.e.,* data generated by the sensor(s). The system may also include a user interface to allow an end user entering a pollinator steering strategy, for instance, in the form of a programming language or a visual programming interface. The system also includes at least one processor configured to implement lighting scenarios or patterns on lamp fixtures aimed at attracting, retaining, stimulating and repulsing pollinators. In some embodiments, the processor is configured to use the sensor data as an input to measure pollinators' response to the light patterns and alter steering strategy accordingly. In these embodiments, the lamps fixtures, sensor(s) and controller(s) are in data communication, using either wired connections or wireless connections. It should be noted that the expression "data communication" may refer to any types of direct connection and/or indirect connection. For example, the communication between the elements may be achieved through direct communication such as a wired connection or via a network allowing data communication between devices or components of a network capable of receiving and/or sending data, which may include, to name a few, publicly accessible networks of linked networks, possibly operated by various distinct parties, such as the Internet, private networks (PN), personal area networks (PAN), local area networks (LAN), wide area networks (WAN), cable networks, satellite networks, cellular telephone networks and the like, or any combinations thereof.

Each zone may include one or more rows of plants or crops, and different lighting scenario can be used in each zone. The horticultural structure includes at least two zones, but it would have been readily understood that the number of zones, and their respective configuration may vary.

In some embodiments, the first and second horticultural light sources may be embodied by a lamp similar to the lighting system described in WO 2016119063, the content of which is incorporated herein by reference.

The controller(s) are configured to send illumination instructions to one of the horticultural light sources, such that the corresponding horticultural light source is driven according to a lighting scenario to meet the lighting conditions of the plants or crops. In some embodiments, each horticultural light source includes a dedicated controller, and each dedicated controller is operatively connected to a user interface. The user interface may be configured to select one of horticultural light source, receive information about the illumination sequence or the lighting scenario being provided by the selected horticultural light source during portion(s) of a lighting scenario and/or send illumination instructions to the selected horticultural light source. As such, the user interface may be in data communication with each dedicated controller of the horticultural light sources through a corresponding communication channel. Of note, the dedicated controller of each horticultural light source may communicate with the user interface over a network. The term "network", synonyms and derivatives thereof are used to refer to any classes of network, which includes publicly accessible networks of linked networks, possibly operated by various distinct parties, such as the Internet, private networks, personal area networks, local area networks, wide area networks, cable networks, satellite networks, cellular telephone networks or any combinations thereof.

In some embodiments, the user interface may be a graphical user interface. In some embodiments, the user interface may be a conversational interface, which may rely on text-activated commands and/or voice-activated commands. The user interface is operatively connected to at least one of the horticultural lighting sources through at least one controller to allow a user to interact with the horticultural lighting sources. In some embodiments, the user interface may be displayed on a display or a screen. In some embodiments, the graphical user interface may be part of a web-based application that may be accessed and displayed using a computing device connected to the Internet or any types of networks. The user interface may also be configured to illustrate the paths followed by the pollinators, which may help identifying optimal lighting conditions for the plants or crops. The user interface can be used to create corridors to be followed by the pollinators, so that the pollinators follow an optimized pollination path and return back to their hives at the end of the day.

In some embodiments, the system may include a memory, or may include or be connected to a database (*e.g.,* through the controller) to store past lighting scenarios or illumination conditions of the plants or crops, calibration data, other relevant data, as well as the impacts of these parameters on the behavior of the pollinators. In some embodiments, the lighting scenarios may be provided in a dataset including a plurality of sets of control parameters for the horticultural light sources. Each set of control parameters may be associated to an illumination state of the horticultural light source, and associated with the impact on the pollination process. The dataset may be stored as a relational database and may have a database format commonly used in the art, such as, for example and without being limitative, CSV/SCSV, SQL, NoSQL, Graph DB, Object Oriented DB, Tabular DB, Large Language Model (LLM), or the like. The dataset may comprise textual information, numeral information, time information, date information, image information, and any combinations thereof. In the context of controlling horticultural light, the control parameters may include the different driving parameters of the horticultural light.

The system having been described may be implemented in computer programs executed on programmable computers. A programmable computer generally includes at least a processor and a data storage system that may include volatile and non-volatile memory and/or storage elements. The programmable computer may be a programmable logic unit, a mainframe computer, server, and personal computer, cloud-based program or system, laptop, personal data assistance, cellular telephone, smartphone, wearable device, tablet device, virtual reality devices, smart display devices, set-top box, video game console, portable video game devices, or virtual reality device. In some embodiments, the systems and methods may be provided as a plug-in. In some embodiments, one or more components of the system having been described may be provided as a plug-in. The expression "plug-in" herein refers to a software component adding a predetermined feature or functionality to the system. Providing the different modules as plug-ins may be associated with some benefits, such as, for example and without being limitative, adaptability, modularity and flexibility.

Of note, the computer programs may be implemented in a high-level procedural or object-oriented programming and/or scripting language to communicate with a computer system. The programs could alternatively be implemented in assembly or machine language, if desired. In these implementations, the language may be a compiled or interpreted language. The computer programs are generally stored on a storage media or a device readable by a general or special purpose programmable computer for configuring and operating the computer when the storage media or device is read by the computer to perform the procedures described herein. In some embodiments, the systems may be embedded within an operating system running on the programmable computer.

In accordance with one aspect, there is provided a method for assisting pollination within a horticultural structure.

The method includes a step of defining a first zone within the horticultural structure. The first zone is associated with a first horticultural light source, and the first horticultural light source is configured to illuminate a first group of plants or crops according to a lighting scenario. The lighting scenario has optical properties based on a targeted photosynthetic growth of the first group of plants or crops.

The method includes a step of defining a second zone within the horticultural structure. The second zone is associated with a second horticultural light source, and the second horticultural light source is configured to illuminate a second group of plants or crops according to a supplemented lighting scenario. The supplemented lighting scenario has at least one expected pollination-enhancing feature.

The method includes a step of, in the first zone, illuminating the first group of plants or crops with the first horticultural light source according to the lighting scenario.

The method includes a step of, in the second zone, illuminating the second group of plants or crops with the second horticultural light source according to the supplemented lighting scenario.

In some embodiments, the method may include monitoring a distribution of pollinators within the first zone and the second zone to determine whether said at least one expected pollination-enhancing feature affects the distribution of pollinators within the second zone in comparison with the first zone.

In some embodiments, defining the control zone and the test zone may include creating the zones, delimiting their limits, adjusting their dimensions, and the like. These steps may be carried out through the user interface. For example, the user interface may be configured to display a representation of the horticultural structure and the horticultural light sources, interact with the representation of the horticultural structure (and components). The user interface may further be configured to create, delete, duplicate, replicate, edit and/or reorganize the zones, based on the plants or crops present in the horticultural structure and their needs.

In some embodiments, the method may include monitoring or tracking the lighting scenarios or recipes according to which the horticultural lighting sources are driven to provide information on the past, actual and/or forecasted lighting scenarios. In some embodiments, the method may include editing or adjusting the lighting scenario based on the correlation between the illumination conditions during the lighting scenarios (*i.e.,* control lighting scenario and test lighting scenario) and their impacts on the pollination process.

In accordance with another aspect of the present description, there is provided a non-transitory computer readable storage medium having stored thereon computer executable instructions that, when executed by a processor, cause the processor to perform the methods that have been previously described. The non-transitory computer storage medium can be integrated to the systems or assemblies that have been described in the present description. The non-transitory computer storage medium could otherwise be operatively connected with the systems or assemblies. In the present description, the terms "computer readable storage medium" and "computer readable memory" are intended to refer to a non-transitory and tangible computer product that can store and communicate executable instructions for the implementation of various steps of the method disclosed herein. The computer readable memory can be any computer data storage device or assembly of such devices, including random-access memory (RAM), dynamic RAM, read-only memory (ROM), magnetic storage devices such as hard disk drives, solid state drives, floppy disks and magnetic tape, optical storage devices such as compact discs (CDs or CDROMs), digital video discs (DVD) and Blu-Ray^{™} discs; flash drive memory, and/or other non-transitory memory technologies. A plurality of such storage devices may be provided, as can be understood by those skilled in the art. The computer readable memory may be associated with, coupled to, or included in a computer or processor configured to execute instructions contained in a computer program stored in the computer readable memory and relating to various functions associated with the computer.

### Examples of Assisting Pollination

Now that some embodiments of the technology have been described, some nonlimitative examples illustrating potential implementations of the techniques for assisting pollination will be presented. It should be noted that these examples serve an illustrative purpose only and should therefore not be considered limitative.

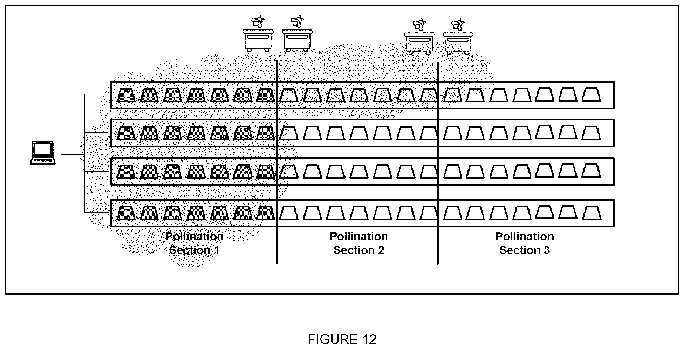

According to a first example, a grower may select bumblebees to facilitate or assist the pollination process within the horticultural structure, *i.e.,* in the production growth environment. In the context of this first example, the controlled agriculture environment (*i.e.,* the horticultural structure) is divided into three virtual sections, as represented in Figure 12 These virtual sections may be defined or created through the user interface or provided as an input from another system. During the growing process, or at least one plant growth stage of the growing process, the grow lights are positioned and oriented to illuminate the plants or crops, so that they can achieve the planned, desired, determined, predetermined or required photosynthetic growth. However, as it often happens, the needs of the plants or crops vary or change. This change may occur during one or more stages of the growing process. For instance, at the pollination stage, the plants or crops should be pollinated, which is typically facilitated with pollinators or pollinating agents. At least one optical property of the light emitted by the horticultural light sources may be adjusted in a determined virtual section, so that the horticultural light sources emit a pollination illumination pattern (sometimes referred to as a "pollination-enhancing spectral feature"), which may include, for example and without being limitative, supplementing the initial light with a spectral peak or band in a range extending from about 375 nm to about 475 nm, in combination with the original scenario delivering the photosynthetic light spectrum. The un-treated sections are illuminated with a strictly photosynthetic light spectrum (*i.e.,* the scenario having optical properties based on the targeted photosynthetic growth of the plants or crops). The enhanced spectrum (*i.e.,* the supplemented lighting scenario) rendered above the concentration section favors or promotes attraction of bumblebees towards the targeted section and stimulates their pollination activity there, while having the optical properties allowing to achieve the targeted photosynthetic growth of the plants or crops. The process can be repeated independently for all sections, thus ensuring an optimal pollination coverage across the horticultural structure.

According to a second example, a greenhouse growth facility is equipped with both traditional high-pressure sodium (HPS) lamps and a network or pool of lamps having variable spectrum capabilities. The greenhouse relies on bumblebees for pollination within this controlled environment. In the context of this second example, it was observed that, during the winter, when the HPS lamps are used, bumblebees have difficulty returning to the hives before sunset, which results in important losses. It was determined that, towards the end of the afternoon, relatively high yellow and red components of the light emitted by the HPS lamps, as well as the relatively low blue, green and UV components of the light emitted by the HPS lamps, combined with the environmental winter illumination conditions were confusing to the bees, and caused a delay between the moment at which the bumblebees should start returning to the hive and the moment at which the bumblebees actually start returning to the hive. It was observed that bumblebees need the blue, green and UV components or portions of the electromagnetic spectrum for orientation and foraging, and that bumblebees do not see in the red portion of the electromagnetic spectrum. As a palliative solution to this situation, the greenhouse operator has implemented a strategy to facilitate guiding the orientation of the bumblebees toward their hives. The pollination window starts around 9 am, when enough natural sunlight is present in the greenhouse for bumblebees to orientate themselves. In the context of the plants or crops of the second example, the pollination windows last for about five hours, until sunlight diminishes to a point where the flowers close. The strategy starts after the pollination period: a subset of variable spectrum light is used to form a corridor of lamps irradiating the plants or crops according to an illumination pattern leading the way towards the hives. The spectrum of the light irradiating that corridor can be supplemented with blue, green and/or UV portions or bands of the electromagnetic spectrum to attract bees within or near the corridor. As the strategy evolves, supplemental variable spectrum lights are turned off in a pattern designed to push bumblebees towards the hives. The whole process goes on for about two hours, when it is assumed that all bumblebees have been able to reach their respective hives.

Several alternative embodiments and examples have been described and illustrated herein. The embodiments described above are intended to be exemplary only. A person skilled in the art would appreciate the features of the individual embodiments, and the possible combinations and variations of the components. A person skilled in the art would further appreciate that any of the embodiments could be provided in any combination with the other embodiments disclosed herein. The present examples and embodiments, therefore, are to be considered in all respects as illustrative and not restrictive. Accordingly, while specific embodiments have been illustrated and described, numerous modifications come to mind without significantly departing from the present disclosure and the appended claims.

## Claims

1. A method for determining pollination illumination patterns within a horticultural structure, the method comprising:
defining a control zone within the horticultural structure, the control zone being associated with a first horticultural light source, the first horticultural light source being configured to illuminate a control plant or crop according to a control lighting scenario;
defining a test zone within the horticultural structure, the test zone being associated with a second horticultural light source, the second horticultural light source being configured to illuminate a test plant or crop according to a test lighting scenario;
in the control zone, illuminating the control plant or crop with the first horticultural light source according to the control lighting scenario;
in the test zone, illuminating the test plant or crop with the second horticultural light source according to the test lighting scenario in the test zone; and
following a release of pollinators within the horticultural structure, determining whether the test lighting scenario impacts a pollination-related property of the test plant or crop with respect to a pollination-related property of the control plant or crop.

2. The method of claim 1, wherein defining the control zone comprises creating the control zone, delimiting the limits of the control zone and/or adjusting the dimensions of the control zone.

3. The method of claim 1 or 2, wherein defining the test zone comprises creating the test zone, delimiting the limits of the test zone and/or adjusting the dimensions of the test zone.

4. The method of any one of claims 1 to 3, further comprising monitoring at least one of the control lighting scenario and the test lighting scenario to collect information on past, actual and/or forecasted lighting scenarios.

5. The method of claim 4, further comprising correlating the information on the past, actual and/or forecasted lighting scenarios with the pollination-related property of the test plant or crop and/or the pollination-related property of the control plant or crop.

6. A system for determining pollination illumination patterns within a horticultural structure, the system comprising:
a first horticultural light source configured to illuminate a control plant or crop according to a control lighting scenario;
a second horticultural light source configured to illuminate a test plant or crop according to a test lighting scenario;
at least one sensor configured to monitor a pollination-related property of the test plant or crop and a pollination-related property of the control plant or crop; and
at least one controller in data communication with the first horticultural light source, the second horticultural light source and said at least one sensor, said at least one controller being configured for:
defining a control zone within the horticultural structure, the control zone being associated with a first horticultural light source;
defining a test zone within the horticultural structure, the test zone being associated with a second horticultural light source;
sending first illumination instructions to the first horticultural light source to irradiate the control plant or crop according to the control lighting scenario in the control zone;
sending second illumination instructions to the second horticultural light source to irradiate the test plant or crop according to the test lighting scenario in the test zone; and
following a release of pollinators within the horticultural structure, determining whether the test lighting scenario impacts a pollination-related property of the test plant or crop with respect to a pollination-related property of the control plant or crop.

7. The system of claim 6, further comprising a first sensor associated with the control plant or crop to track the pollination-related property of the control plant or crops.

8. The system of claim 7, further comprising a second sensor associated with the test plant or crop to track the pollination-related property of the test plant or crop.

9. The system of claim 7 or 8, wherein at least one of the first sensor and the second sensor is a camera or a photodetector.

10. The system of any one of claims 7 to 9, wherein the first sensor and the second sensor are each configured to detect characteristics associated with flying insects.

11. The system of claim 10, wherein the characteristics are at least one of a species of the flying insects, a sex of the flying insects, a physiological state of the flying insects and a relative position of the flying insects with respect to the first horticultural light source and/or the second horticultural source.

12. The system of any one of claims 7 to 11, wherein the first sensor and the second sensor are each configured to detect a presence or an absence of the flying insects.

13. The system of any one of claims 6 to 12, further comprising a user interface allowing an end user to enter a pattern discovery strategy as a programming language or a visual programming interface.

14. The system of any one of claims 6 to 13, wherein each one of the control zone and the test zone comprises one or more rows of plants or crops.

15. The system of any one of claims 6 to 14, wherein the first horticultural source and the second horticultural source each comprise a dedicated controller.
